# EUROPEAN PATENT SPECIFICATION

(11) **EP 4 066 794 B1**
(45) Date of publication and mention of the grant of the patent: **21.05.2025**
(21) Application number: 20908312.0
(22) Date of filing: 10.09.2020
(51) Int. Cl.: A61F 2/82, A61F 2/90, A61M 25/09

(54) **KNITTED STENT AND KNITTED STENT SYSTEM**
GESTRICKTER STENT UND GESTRICKTES STENTSYSTEM
ENDOPROTHÈSE TRICOTÉE ET SYSTÈME D'ENDOPROTHÈSE TRICOTÉE

(30) Priority: 25.12.2019 CN 201911360870
(43) Date of publication of application: 05.10.2022
(73) Proprietor: Shanghai VasoLutions MedTech Co., Ltd., Shanghai 201318 (CN)
(72) Inventor: ZHU, Qing, Shanghai 201318 (CN); ZHU, Yongfeng, Shanghai 201318 (CN); LIU, Mengqin, Shanghai 201318 (CN)
(74) Representative: Marks & Clerk LLP
(86) International application number: PCT/CN2020/114612
(87) International publication number: WO 2021/128953

(56) References cited:
- WO-A1-2009/048813
- WO-A1-2013/126197
- CN-A- 101 827 567
- CN-A- 101 827 567
- CN-A- 103 108 614
- CN-A- 106 963 525
- CN-A- 109 394 399
- US-A1- 2005 266 039
- US-A1- 2007 244 520
- US-A1- 2009 132 031
- US-A1- 2012 067 454
- US-A1- 2013 197 626

## Description

### TECHNICAL FIELD

The present invention relates to the field of medical devices and, more particularly, to a knitted stent and a knitted stent system.

### BACKGROUND

Peripheral artery disease (PAD) is characterized by atherosclerosis of peripheral arteries, associated with a risk of myocardial infarction, cerebral stroke and vascular death and prevalent in the elderly population. With the development of vascular interventional technology, mainstream surgical PAD treatment has been gradually transitioning from open surgery to endovascular interventional surgery.

Typical interventional surgical procedures for PAD treatment are balloon dilation and stenting procedures. However, as satisfactory long-term outcomes are unachievable only by balloon dilation for most patients, stenting is considered critical for PAD treatment. At present, stents used in PAD treatment are mainly self-expanding ones.

Self-expanding stents can be categorized into laser-cut stents and knitted stents. No matter which of these types is used in a PAD treatment procedure, it is inevitable for the stent to stimulate the inner wall, and cause endothelial hyperplasia, of the blood vessel where it is deployed, which may ultimately develop to vascular restenosis. Currently, this problem is overcome mainly by using the drug-loaded stents. Most existing drug-loaded stents choose laser-cut stents as the drug carriers, in which grooves are engraved and drugs are deposited in the grooves. However, the laser-cut stents are insufficient in geometric compliance and fatigue resistance and tend to break when the blood vessel anatomy requires significant deformation. Knitted stents have good mechanical properties, but forming grooves or holes for receiving drugs in fine wires from which they are knitted is costly or even infeasible. Although it is also contemplated to obtain drug-loaded stents by directly spraying drugs onto wires of knitted stents, the so-formed drug-containing films can easily peel off and tend to not allow sustained drug release.

WO2009/4881381 describes a system for treating a vascular condition including a catheter and a stent disposed on the catheter. The stent includes tubing having a wall defining a central lumen and a plurality of holes. The system further includes a therapeutic agent disposed within the central lumen of the tubing. A method of manufacturing a therapeutic agent is also described. US0213/197626 A1 describes a stent formed from a wire that in cross section includes an outer member having a lumen and a radiopaque core member partially filling the lumen. A substance for elution through openings formed through the outer member fills the portion of the lumen not filled by the radiopaque core member. A method of forming the stent is also described.

### SUMMARY OF THE INVENTION

In order to overcome the above-described problem of difficulty in drug loading with conventional knitted stents, it is an objective of the present invention to provide a knitted stent and a knitted stent system.

The above objective is attained by a knitted stent according to the present invention, which includes a wire spirally coiled about an axis in an interlaced manner, the wire including at least one elastic metal tube each defining a first lumen and having a wall in which release holes in communication with the first lumen are formed. A liner core is disposed within the first lumen so as to exactly fill the first lumen. An outer surface of the liner core is brought into tight contact with an inner wall of the first lumen so that the outer surface of the liner core and the walls of the release holes together delimit receptacles.

Optionally, the first lumen may be filled with a drug or a mixture of a drug and a drug carrier.

Optionally, the receptacle may be filled with a drug or a mixture of a drug and a drug carrier.

Optionally, the receptacle may have a depth of from 0.02 mm to 0.2 mm.

Optionally, an outer surface of each elastic metal tube may be provided with an absorbable film, the film covering the release holes.

Optionally, the wire may be consisted of the at least one elastic metal tube.

Optionally, the wire may further include a solid metal wire.

Optionally, the release holes may be provided on an outer surface of the knitted stent.

Optionally, the release holes may be distributed at a density that is higher around both ends of the knitted stent than in a middle section of the knitted stent.

Optionally, the knitted stent may further include a connection portion arranged at an end of the knitted stent, and wherein each elastic metal tube is connected to a further wire via the connection portion.

Optionally, the wire may include at least two elastic metal tubes, wherein one of the elastic metal tubes is connected to another one of the elastic metal tubes via the connection portion.

Optionally, the wire may further include a solid metal wire, and wherein when any elastic metal tube is connected to the solid metal wire, the connection portion is formed by a bent section of the solid metal wire.

Optionally, each elastic metal tube may be made essentially of a radiopaque metal, and/or the connection portion may be made essentially of a radiopaque metal.

Optionally, each elastic metal tube may have a circular or elongated radial cross-section.

Optionally, when each elastic metal tube has a circular radial cross-section, the elastic metal tube may have an outer diameter of from 0.1 mm to 0.5 mm.

Optionally, when the first lumen of each elastic metal tube has a circular radial cross-section, the first lumen may have a diameter of from 0.05 mm to 0.45 mm.

Optionally, the release holes may have a diameter of from 0.05 mm to 0.2 mm.

The above objective is also attained by a knitted stent system according to the present invention, which includes a delivery guidewire, a delivery catheter and a knitted stent as defined in any of the preceding paragraphs. The delivery guidewire is configured to load the knitted stent thereon, and the delivery catheter defines a second lumen extending through the delivery catheter along an axial direction thereof. The second lumen is configured to accommodate both the delivery guidewire and the knitted stent.

Compared with the prior art, the knitted stent and the knitted stent system of the present invention offer the following advantages:
First, the wire(s) in the knitted stent is/are spirally coiled about an axis in an interlaced manner and include(s) at least one elastic metal tube each defining a first lumen and having a wall in which release holes in communication with the first lumen are formed. Additionally, a drug or a mixture of a drug and a drug carrier can be filled in the first lumen and released from the release holes. This solves the problem of difficulty in loading drugs arising from the use of conventional knitted stents.
Second, a liner core may be so arranged in the first lumen as to at least partially fill the first lumen. In this way, an outer surface of the liner core and walls of the release holes together delimit a receptacle in which a drug or a mixture of a drug and a drug carrier can be filled. As such, through appropriately designing the liner core's dimensions, it is not only possible to adjust a drug loading amount of the knitted stent but also to further enhance fatigue resistance and radial support of the knitted stent.

### BRIEF DESCRIPTION OF THE DRAWINGS

Fig. 1 is a structural schematic of a knitted stent according to an embodiment of the present invention, which is knitted from wire(s) each implemented as an elastic metal tube with circular release holes.
Fig. 2 is a structural schematic of a knitted stent according to an embodiment of the present invention, in which release holes are oblong.
Fig. 3 schematically illustrates release holes in an elastic metal tube in a knitted stent according to an embodiment of the present invention.
Fig. 4 schematically illustrates an elastic metal tube in a knitted stent according to an embodiment of the present invention, in which a liner core is filled.
Fig. 5 is a partial schematic view of a knitted stent according to an embodiment of the present invention.
Fig. 6 is a structural schematic of a knitted stent according to an embodiment of the present invention, which is knitted from wires including elastic metal tube(s) and solid metal wire(s).

### List of Reference Numerals

100-Knitted Stent
110-Elastic Metal Tube
111-Release Hole
112-Liner Core
120-Connection Portion
130-Metal Wire

### DETAILED DESCRIPTION

The core idea of the present invention is to provide a knitted stent that defines a drug receptacle in which a drug can be filled and can thus be used to produce a drug-loaded stent.

The stent is knitted from wire(s) spirally coiled about an axis in an interlaced manner. The wire(s) include(s) at least one elastic metal tube each defining a first lumen and having a wall in which release holes in communication with the first lumen are formed.

According to embodiments of the present invention, the elastic metal tube(s) can be formed using an existing non-expensive technique for fabricating capillaries, and forming the holes in the tube(s) can be accomplished using an established technique. All of these make it possible to produce a drug-loaded stent from the knitted stent.

Objects, advantages and features of the present invention will become apparent upon reading the following more detailed description with reference to the accompanying drawings. Note that the figures are provided in a very simplified form not necessarily presented to scale, with their only intention to facilitate convenience and clarity in explaining embodiments disclosed herein.

As used herein, the singular terms "a", "an" and "the" include plural referents, while the term "plurality" means two or more, unless the context clearly dictates otherwise. As used herein, the term "or" is generally employed in the sense including "and/or", unless the context clearly dictates otherwise, and the terms "mount", "couple" and "connect" in their various forms should be interpreted in a broad sense. For example, a "connection" may refer to a fixed connection, a detachable connection, or an integrated connection, or to a mechanical connection or an electrical connection, or to a direct connection or an indirect connection with one or more elements interposed between the connected ones, or to mutual communication between the interiors of two elements or interaction between two elements. A person of ordinary skill in the art would be able to understand the various uses of these terms based upon their context, and identical or similar reference numerals will be used throughout the drawings to identify identical or similar items.

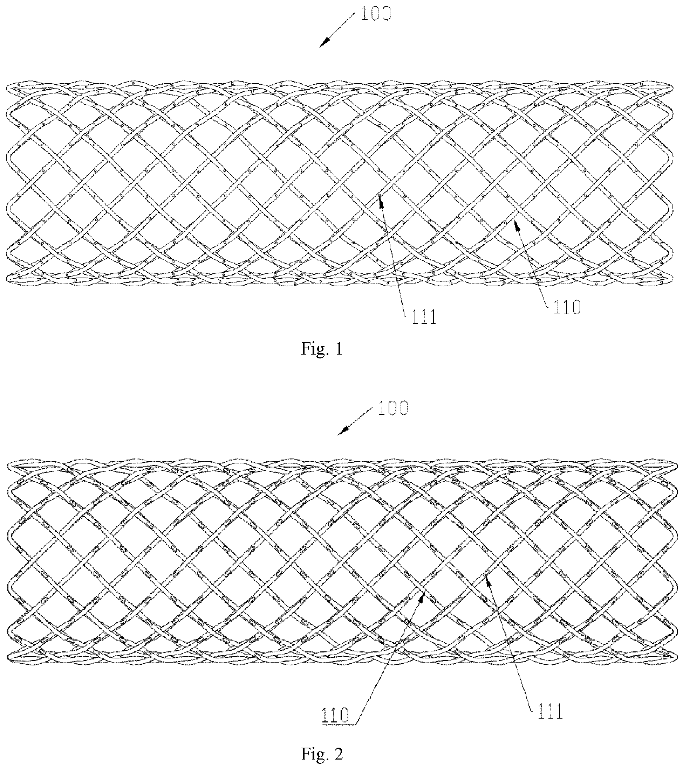

Fig. 1 schematically illustrates the structure of a knitted stent according to an embodiment of the present invention. As shown in Fig. 1, the stent 100 is knitted from wire(s) which is/are spirally coiled about an axis in an interlaced manner. In generally, the number of the wire(s) may be greater than one. In this case, the wires include at least one elastic metal tube 110. Each elastic metal tube 110 defines a first lumen, with release holes 111 in communication with the first lumen being formed in a wall of the elastic metal tube 110.

In this embodiment, each elastic metal tube 110 is spiral without sharp bends and thus free of the problem of tending to break, thereby ensuring good performance of the knitted stent 100 during use. Moreover, the first lumen of each elastic metal tube 110 can serve as a drug receptacle, which when filled with a drug makes the knitted stent 100 a drug-loaded stent. This circumvents the problem of poor geometric compliance and fatigue resistance arising from the use of conventional drug-loaded stents. Each elastic metal tube 110 may be fabricated as a hollow capillary at a cost affordable for most manufacturers.

The first lumen may be filled with a drug or a mixture of a drug and a drug carrier. The drug may be, for example, paclitaxel, rapamycin or another agent that can inhibit vascular endothelial hyperplasia. In this way, when the drug-loaded stent is deployed in a diseased blood vessel, the drug may be released from the release holes 111 and acts on the blood vessel to prevent its restenosis. The drug may be mixed with the drug carrier before they are filled in the first lumen. The drug carrier may facilitate the drug's sustained release by regulating the drug's release rate. For example, the drug may be mixed with a film solution and then filled into the first lumen. The carrier for loading the drug may be a biologically stable polymer. When the carrier is degradable, the regulation of the drug's release rate can be accomplished by adjusting the carrier's degradation rate.

Preferably, the release holes 111 are arranged across an outer surface of the knitted stent 100 so that the released drug will come into direct contact with an inner wall of the blood vessel and act on the inner wall of the blood vessel without migration into the vascular lumen. This arrangement is advantageous in that the drug can effectively act to inhibit hyperplasia on the inner wall of the blood vessel while avoiding endothelialization.

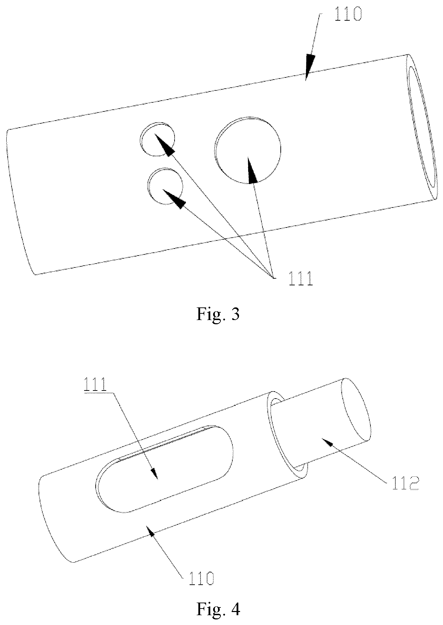

The release holes 111 are arranged to provide paths for the drug's release. Therefore, this embodiment is not limited to any particular shape of the release holes 111. The release holes 111 may be circular (as shown in Fig. 1), oblong (as shown in Figs. 2 and 4), elliptic, diamond-shaped, square or otherwise shaped, depending on the actual needs. In case of circular release holes 111, a diameter of the release holes 111 may be approximately 0.05-0.2 mm. Reference can be made to the area of such circular release holes 111 for information about the area of differently-shaped release holes 111.

In each elastic metal tube 110, the release holes 111 may be distributed along an axial direction of the elastic metal tube 110 so that the drug can be released from various segments of the knitted stent 100. The release holes 111 may be all of the same size or not (as shown in Fig. 3). Moreover, the release holes 111 may be of the same shape or not. In practical treatment scenarios, endothelial hyperplasia has been more observed on the inner wall sections of the diseased blood vessel around both ends of the implanted knitted stent 100. Accordingly, the release holes 111 are preferred to be distributed more densely at end portions of the knitted stent 100 than at a middle section of the knitted stent 100. In this way, the drug can be released at a greater amount at both ends of the knitted stent 100 to enhance the inhibitory effect. In some embodiments, the release holes 111 at the various sections may have different sizes and hence different total opening areas. For example, when the release holes 111 are uniformly distributed in each elastic metal tube 110 along the axial direction of the elastic metal tube 110, those close to the ends of the knitted stent 100 may be arranged to have a greater diameter than that of those at the middle section of the knitted stent 100. Alternatively, in other embodiments, the density of the release holes 111 may vary due to different distances between adjacent release holes 111. For example, when all of the release holes 111 are equally sized, the distance between adjacent release holes 111 may gradually decrease in both directions from the middle section of the knitted stent 100 toward the ends of the knitted stent 100.

Fig. 4 shows a schematic diagram of the structure of a knitted stent according to another embodiment, which is modified essentially in allowing adjustments in a drug loading amount of each elastic metal tube 110. Specifically, a liner core 112 may be added into the first lumen. The liner core 112 may be made essentially of a metallic material. The liner core 112 is equal to the first lumen in both length and radial dimension. In this case, when disposed within the first lumen, the liner core 112 will exactly fill the first lumen. As a result, the outer surface of the liner core 112 is brought into tight contact with the inner wall of the first lumen and delimits, together with the walls of the release holes 111, the receptacle for holding the drug. In other words, through appropriately designing the length and radial dimension of the liner core 112, the drug receptacle can be adapted to allow a desirable drug loading amount of each elastic metal tube 110. When delimiting the drug receptacle with the liner core 112 disposed in the first lumen, it is preferred that the receptacle has a depth between 0.02 mm and 0.2 mm. In addition, arranging the liner core 112 in the first lumen can result in enhanced fatigue resistance of each elastic metal tube 110 and higher radial support that the knitted stent 100 can provide, thus leading to improved mechanical properties of the knitted stent 100.

It will be appreciated that the knitted stent 100 may include a plurality of elastic metal tubes 110. In practical use, the liner core 112 may be disposed in the first lumen of some of the elastic metal tubes 110 as desired, or the liner core 112 may be disposed in the first lumen of all the elastic metal tubes 110 as desired.

Further, each elastic metal tube 110 may be provided over its outer surface with an absorbable film (not shown) which at least covers the release holes 111. The film is provided to prevent any loss of the drug before the knitted stent 100 is implanted into the blood vessel. When the knitted stent 100 is deployed in the blood vessel, the film can be absorbed in the blood vessel so that the release holes 111 are exposed, allowing release of the drug. The absorbable film can be polylactic acid, polyglycolic acid, a lactic acidglycolic acid copolymer or another synthetic polymer for medical use.

For the sake of easier fabrication, each elastic metal tube 110 and the first lumen thereof are preferred to both have a circular radial cross-section. In this case, the elastic metal tube 110 may have an outer diameter of 0.1-0.5 mm and an inner diameter (i.e., a diameter of the first lumen) ranging from 0.05 mm to 0.45 mm. However, for the sake of good performance of the knitted stent 100 during use, each elastic metal tube 110 is preferred to have an elongated cross-section such as elliptic or oblong, which allows the knitted stent 100 to have a reduced wall thickness. The elongated or otherwise shaped radial cross-section may be equivalent to that of the circular radial cross-section.

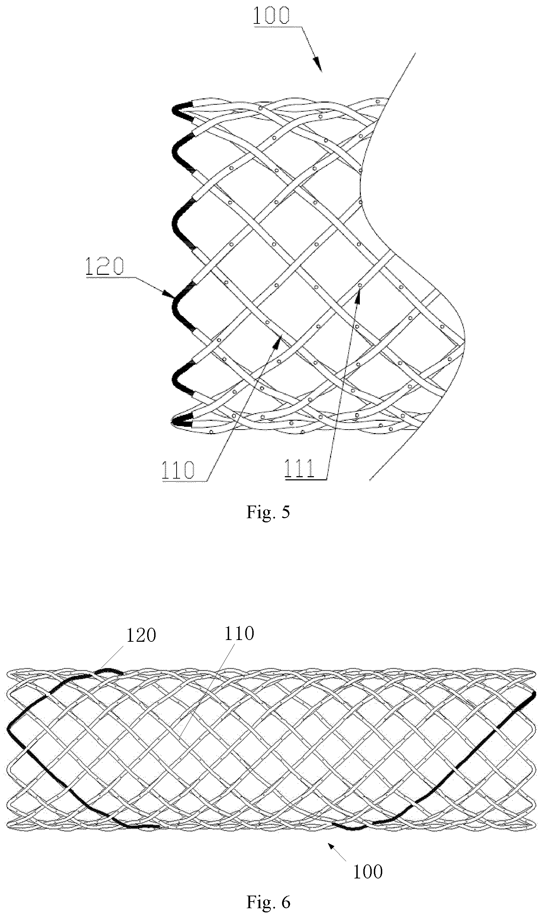

Further, since it is undesirable for any elastic metal tube 110 to have sharp bends (which may augment the tendency of the elastic metal tube 110 to break), as shown in Fig. 5, at end(s) of the knitted stent 100, each elastic metal tube 110 is connected to another wire by a connection portion 120. In this embodiment, each wire is an elastic metal tube 110, and each connection portion 120 is a V-shaped or curved structure made of a metal. Each connection portion 120 may have two ends, each of which may be welded to one end of an elastic metal tube 110. In this way, all the elastic metal tube(s) 110 can be interconnected so as to immunize the knitted stent 100 from an issue of slackness.

Optionally, at least one of the elastic metal tube(s) 110, the liner core(s) 112 and the connection portion 120 may be made of a radiopaque metal to impart to the knitted stent 100 radiopacity, which is helpful in the positioning of the knitted stent 100 in the blood vessel. Examples of the radiopaque metal may include, but are not limited to, tantalum, platinum, gold, platinum-tungsten alloys or platinum-iridium alloys.

In other embodiments, as shown in Fig. 6, the wire(s) from which the stent 100 is knitted may further include solid metal wire(s) 130. In this case, the metal wire(s) 130 may be interlaced with the elastic metal tube(s) 110 to form the knitted stent 100. In this way, the knitted stent 100 can be fabricated at a lower cost. A ratio of the number of the metal wire(s) 130 to that of the elastic metal tube(s) 110 may depend on a drug loading amount of the knitted stent 100, and this embodiment is not limited any particular value thereof.

Further, in this embodiment, in case of the wire(s) including at least two elastic metal tubes 110, one of the elastic metal tubes 110 may be connected to another or the other elastic metal tube 110 by a connection portion 120. Alternatively, one of the elastic metal tubes 110 may be connected to a metal wire 130. In the latter case, a bent end portion of the metal wire 130 may serve as a connection portion 120. Of course, the metal wire(s) 130 may also be formed of a radiopaque metal.

On the basis of the knitted stent 100 as defined above, in embodiments of the present invention, there is also provided a knitted stent system include a delivery guidewire, a delivery catheter and the knitted stent. The delivery guidewire is configured to deliver the knitted stent (by disposing the knitted stent over the delivery guidewire), and the delivery catheter defines a second lumen, which extends through the catheter along an axial direction thereof and is configured to accommodate both the delivery guidewire and the knitted stent.

The knitted stent according to the present invention is knitted from wire(s) including elastic metal tube(s) each defining a first lumen that can serve as a drug receptacle for the knitted stent. In this way, the knitted stent can be used as a base of a drug-loaded stent, and the problem of poor mechanical properties arising from the use of conventional drug-loaded stents is solved.

Although the present invention has been disclosed above, it is not limited to the above disclosure. Those skilled in the art can make various modifications and variations to the present invention without departing from the scope thereof, as defined by the claims.

Accordingly, the invention is intended to embrace all such modifications and variations if they fall within the scope of the appended.

## Claims

1. A knitted stent (100) comprising a wire spirally coiled about an axis in an interlaced manner, the wire including at least one elastic metal tube (110), each elastic metal tube (110) defining a first lumen and having a wall in which release holes (111) in communication with the first lumen are formed, **characterized in that**:
a liner core (112) is disposed within the first lumen so as to exactly fill the first lumen, an outer surface of the liner core (112) is brought into tight contact with an inner wall of the first lumen so that the outer surface of the liner core (112) and walls of the release holes (111) together delimit receptacles.

2. The knitted stent (100) of claim 1, wherein the first lumen is filled with a drug or a mixture of a drug and a drug carrier.

3. The knitted stent (100) of claim 1, wherein the release holes (111) are distributed at a density that is higher around both ends of the knitted stent (100) than in a middle section of the knitted stent (100).

4. The knitted stent (100) of claim 1, wherein the receptacle is filled with a drug or a mixture of a drug and a drug carrier; and/or
wherein the receptacle has a depth of from 0.02 mm to 0.2 mm.

5. The knitted stent (100) of claim 1 or 2, wherein an outer surface of each elastic metal tube (110) is provided with an absorbable film, the film covering the release holes (111).

6. The knitted stent (100) of claim 1, wherein the wire is consisted of the at least one elastic metal tube (110); and/or
wherein the wire further includes a solid metal wire (130).

7. The knitted stent (100) of claim 1, wherein the release holes (111) are provided on an outer surface of the knitted stent (100).

8. The knitted stent (100) of claim 1, further comprising a connection portion (120) arranged at an end of the knitted stent, and wherein each elastic metal tube (110) is connected to a further wire via the connection portion (120).

9. The knitted stent (100) of claim 8, wherein the wire includes at least two elastic metal tubes, wherein one of the elastic metal tubes is connected to another one of the elastic metal tubes via the connection portion (120); and/or
wherein the wire further includes a solid metal wire (130), and wherein when any elastic metal tube is connected to the solid metal wire (130), the connection portion (120) is formed by a bent section of the solid metal wire (130).

10. The knitted stent (100) of claim 9, wherein each elastic metal tube (110) is made essentially of a radiopaque metal, and/or wherein the connection portion (120) is made essentially of a radiopaque metal.

11. The knitted stent (100) of claim 1, wherein each elastic metal tube (110) has a circular or elongated radial cross-section.

12. The knitted stent (100) of claim 11, wherein when each elastic metal tube has a circular radial cross-section, the elastic metal tube (110) has an outer diameter of from 0.1 mm to 0.5 mm; and/or
wherein when the first lumen of each elastic metal tube (110) has a circular radial cross-section, the first lumen has a diameter of from 0.05 mm to 0.45 mm.

13. The knitted stent (100) of claim 1, wherein each of the release holes (111) has a diameter of from 0.05 mm to 0.2 mm.

14. A knitted stent system comprising a delivery guidewire, a delivery catheter and a knitted stent (100) as defined in any one of claims 1 to 13, the delivery guidewire configured to load the knitted stent (100) thereon, the delivery catheter defining a second lumen extending through the delivery catheter along an axial direction thereof, the second lumen configured to accommodate both the delivery guidewire and the knitted stent (100).

## Patentansprüche

1. Gestrickter Stent (100), umfassend einen Draht, der spiralförmig um eine Achse in einer verflochtenen Weise gewunden ist, wobei der Draht mindestens eine elastische Metallröhre (110) einschließt, wobei jede elastische Metallröhre (110) ein erstes Lumen definiert und eine Wand aufweist, in der Freisetzungslöcher (111) in Kommunikation mit dem ersten Lumen gebildet sind, **dadurch gekennzeichnet, dass**
ein Auskleidungskern (112) in dem ersten Lumen so angeordnet ist, dass er das erste Lumen genau ausfüllt, eine Außenfläche des Auskleidungskerns (112) in engen Kontakt mit einer Innenwand des ersten Lumens gebracht wird, sodass die Außenfläche des Auskleidungskerns (112) und die Wände der Freisetzungslöcher (111) Behälter zusammen begrenzen.

2. Gestrickter Stent (100) nach Anspruch 1, wobei das erste Lumen mit einem Arzneimittel oder einer Mischung aus einem Arzneimittel und einem Arzneimittelträger gefüllt ist.

3. Gestrickter Stent (100) nach Anspruch 1, wobei die Freisetzungslöcher (111) mit einer Dichte verteilt sind, die um beide Enden des gestrickten Stents (100) herum höher ist als in einer mittleren Sektion des gestrickten Stents (100).

4. Gestrickter Stent (100) nach Anspruch 1, wobei der Behälter mit einem Arzneimittel oder einer Mischung aus einem Arzneimittel und einem Arzneimittelträger gefüllt ist; und/oder
wobei der Behälter eine Tiefe von 0,02 mm bis 0,2 mm aufweist.

5. Gestrickter Stent (100) nach Anspruch 1 oder 2, wobei eine Außenfläche jeder elastischen Metallröhre (110) mit einer resorbierbaren Folie versehen ist, wobei die Folie die Freisetzungslöcher (111) bedeckt.

6. Gestrickter Stent (100) nach Anspruch 1, wobei der Draht aus dem mindestens einer elastischen Metallröhre (110) besteht; und/oder
wobei der Draht ferner einen massiven Metalldraht (130) einschließt.

7. Gestrickter Stent (100) nach Anspruch 1, wobei die Freisetzungslöcher (111) auf einer Außenfläche des gestrickten Stents (100) bereitgestellt sind.

8. Gestrickter Stent (100) nach Anspruch 1, ferner umfassend einen Verbindungsabschnitt (120), der an einem Ende des gestrickten Stents angeordnet ist, und wobei jede elastische Metallröhre (110) über den Verbindungsabschnitt (120) mit einem weiteren Draht verbunden ist.

9. Gestrickter Stent (100) nach Anspruch 8, wobei der Draht mindestens zwei elastische Metallröhren einschließt, wobei eine der elastischen Metallröhren mit einer anderen der elastischen Metallröhren über den Verbindungsabschnitt (120) verbunden ist; und/oder
wobei der Draht ferner einen massiven Metalldraht (130) einschließt, und wobei, wenn irgendeine elastische Metallröhre mit dem massiven Metalldraht (130) verbunden ist, der Verbindungsabschnitt (120) durch eine gebogene Sektion des massiven Metalldrahts (130) gebildet wird.

10. Gestrickter Stent (100) nach Anspruch 9, wobei jede elastische Metallröhre (110) im Wesentlichen aus einem röntgendichten Metall besteht und/oder wobei der Verbindungsabschnitt (120) im Wesentlichen aus einem röntgendichten Metall besteht.

11. Gestrickter Stent (100) nach Anspruch 1, wobei jede elastische Metallröhre (110) einen kreisförmigen oder länglichen radialen Querschnitt aufweist.

12. Gestrickter Stent (100) nach Anspruch 11, wobei, wenn jede elastische Metallröhre einen kreisförmigen radialen Querschnitt aufweist, die elastische Metallröhre (110) einen Außendurchmesser von 0,1 mm bis 0,5 mm aufweist; und/oder
wobei, wenn das erste Lumen jeder elastischen Metallröhre (110) einen kreisförmigen radialen Querschnitt aufweist, das erste Lumen einen Durchmesser von 0,05 mm bis 0,45 mm aufweist.

13. Gestrickter Stent (100) nach Anspruch 1, wobei jedes der Freisetzungslöcher (111) einen Durchmesser von 0,05 mm bis 0,2 mm aufweist.

14. Gestricktes Stentsystem, umfassend einen Zuführungs-Führungsdraht, einen Zuführungskatheter und einen gestrickten Stent (100) wie in einem der Ansprüche 1 bis 13 definiert, wobei der Zuführungs-Führungsdraht konfiguriert ist, um den gestrickten Stent darauf zu laden, wobei der Zuführungskatheter ein zweites Lumen definiert, das sich durch den Zuführungskatheter entlang einer axialen Richtung davon erstreckt, wobei das zweite Lumen konfiguriert ist, um sowohl den Zuführungs-Führungsdraht als auch den gestrickten Stent (100) aufzunehmen.

## Revendications

1. Endoprothèse tricotée (100) comprenant un fil enroulé en spirale autour d'un axe de manière entrelacée, le fil incluant au moins un tube métallique élastique (110), chaque tube métallique élastique (110) définissant une première lumière et ayant une paroi dans laquelle sont formés des trous de dégagement (111) en communication avec la première lumière ; **caractérisée en ce que** :
un noyau de revêtement (112) est disposé à l'intérieur de la première lumière de manière à remplir exactement la première lumière, une surface extérieure du noyau de revêtement (112) est mise en contact étroit avec une paroi intérieure de la première lumière de façon que la surface extérieure du noyau de revêtement (112) et les parois des trous de dégagement (111) délimitent ensemble des réceptacles.

2. Endoprothèse tricotée (100) selon la revendication 1, dans laquelle la première lumière est remplie d'un médicament ou d'un mélange d'un médicament et d'un support de médicament.

3. Endoprothèse tricotée (100) selon la revendication 1, dans laquelle les trous de dégagement (111) sont répartis selon une densité qui est plus élevée autour des deux extrémités de l'endoprothèse tricotée (100) que dans une section médiane de l'endoprothèse tricotée (100).

4. Endoprothèse tricotée (100) selon la revendication 1, dans laquelle le réceptacle est rempli d'un médicament ou d'un mélange d'un médicament et d'un support de médicament ; et/ou
dans laquelle le réceptacle a une profondeur allant de 0,02 mm à 0,2 mm.

5. Endoprothèse tricotée (100) selon la revendication 1 ou la revendication 2, dans laquelle une surface extérieure de chaque tube métallique élastique (110) est pourvue d'un film absorbable, le film recouvrant les trous de dégagement (111).

6. Endoprothèse tricotée (100) selon la revendication 1, dans laquelle le fil est constitué dudit au moins un tube métallique élastique (110) ; et/ou
dans laquelle le fil inclut en outre un fil métallique plein (130).

7. Endoprothèse tricotée (100) selon la revendication 1, dans laquelle les trous de dégagement (111) sont prévus sur une surface extérieure de l'endoprothèse tricotée (100).

8. Endoprothèse tricotée (100) selon la revendication 1, comprenant en outre une partie de raccord (120) disposée à une extrémité de l'endoprothèse tricotée, et dans laquelle chaque tube métallique élastique (110) est raccordé à un autre fil par la partie de raccord (120).

9. Endoprothèse tricotée (100) selon la revendication 8, dans laquelle le fil inclut au moins deux tubes métalliques élastiques, l'un des tubes métalliques élastiques étant raccordé à un autre des tubes métalliques élastiques par la partie de raccord (120) ; et/ou
dans laquelle le fil inclut en outre un fil métallique plein (130), et dans laquelle, lorsqu'un quelconque tube métallique élastique est raccordé au fil métallique plein (130), la partie de raccord (120) est formée par une section courbée du fil métallique plein (130).

10. Endoprothèse tricotée (100) selon la revendication 9, dans laquelle chaque tube métallique élastique (110) se compose essentiellement d'un métal radio-opaque, et/ou dans laquelle la partie de raccord (120) se compose essentiellement d'un métal radio-opaque.

11. Endoprothèse tricotée (100) selon la revendication 1, dans laquelle chaque tube métallique élastique (110) a une section transversale radiale circulaire ou allongée.

12. Endoprothèse tricotée (100) selon la revendication 11, dans laquelle, lorsque chaque tube métallique élastique a une section transversale radiale circulaire, le tube métallique élastique (110) a un diamètre extérieur allant de 0,1 mm à 0,5 mm ; et/ou
dans laquelle, lorsque la première lumière de chaque tube métallique élastique (110) a une section transversale radiale circulaire, la première lumière a un diamètre allant de 0,05 mm à 0,45 mm.

13. Endoprothèse tricotée (100) selon la revendication 1, dans laquelle chacun des trous de dégagement (111) a un diamètre allant de 0,05 mm à 0,2 mm.

14. Système d'endoprothèse tricotée comprenant un fil-guide de mise en place, un cathéter de mise en place et une endoprothèse tricotée (100) tel que définie dans l'une quelconque des revendications 1 à 13, le fil-guide de mise en place étant configuré pour charger l'endoprothèse tricotée (100) sur celui-ci, le cathéter de mise en place définissant une seconde lumière s'étendant à travers le cathéter de mise en place le long d'une direction axiale de celui-ci, la seconde lumière étant configurée pour recevoir à la fois le fil-guide de mise en place et l'endoprothèse tricotée (100).
